# EUROPEAN PATENT SPECIFICATION

(11) **EP 2 371 226 B1**
(45) Date of publication and mention of the grant of the patent: **08.03.2017**
(21) Application number: 10756329.8
(22) Date of filing: 23.03.2010
(51) Int. Cl.: C07C 69/67, A23K 20/105, A23K 50/00, A23K 50/75, A23K 50/30

(54) **BILE SALT ADJUVANT FOR ANIMALS FOR IMPROVING FAT UTILIZATION EFFICIENCY IN THE BODIES OF ANIMALS**
GALLENSALZADJUVANS FÜR TIERE ZUR VERBESSERUNG DER FETTNUTZUNGSEFFIZIENZ IN TIERKÖRPERN
ADJUVANT DE SELS BILIAIRES POUR ANIMAUX, DESTINÉ À AMÉLIORER L'EFFICACITÉ D'UTILISATION DES GRAISSES DANS LE CORPS DES ANIMAUX

(30) Priority: 26.03.2009 KR 20090025752
(43) Date of publication of application: 05.10.2011
(73) Proprietor: Yun, Kwan-Sik, Gyeonggi-do 420-100 (KR); Kimin Inc., Seoul 137-897 (KR)
(72) Inventor: YUN, Kwam-Sik, Gyeonggi-do 420-100 (KR)
(74) Representative: Modiano, Micaela Nadia
(86) International application number: PCT/KR2010/001765
(87) International publication number: WO 2010/110574

(56) References cited:
- EP-A2- 0 130 746
- WO-A1-96/13175
- WO-A1-98/09538
- GB-A- 1 581 744
- GB-A- 2 118 017
- KR-A- 20040 032 515
- US-A- 4 804 549
- US-A- 6 063 776
- US-A1- 2005 019 461

## Description

### [Technical Field]

The present invention relates to a bile salt adjuvant for animals which is used for animal feed to improve fat utilization efficiency the bodies of animals and reduce the amount of fat used in the feed and thereby improve production efficiency.

### [Background Art]

The term "animal feed" refers to a substance which provides organic or inorganic nutrients required for sustaining the life of livestock and producing milk, meat, eggs, furs and the like. The animal feed is prepared by mixing a variety of nutrients essential for animals, such as energy, proteins, vitamins and minerals as well as growth promoting agents and disease-preventing agents.

Animal feed performs a variety of functions, for example, provides nutrients essential for survival and growth of livestock, strengthens immune functions, improves qualities of livestock products and enhances livestock barn environments.

In particular, an increase in production efficiency of livestock is carried out by improving livestock barn environments or enhancing animal feed efficiency. A great deal of research has been made to improve animal feed efficiency, for example, by adding a novel composition to a conventional animal feed or varying mix ratio or food supply manner.

For example, KR Patent Laid-open No. 2006-35444 discloses an animal feed and a method for raising animals using the same. This patent suggests an animal feed comprising general animal feed ingredients as well as bamboo charcoal to increase body weight or body weight gain.

EP0130746 A2 discoses the use of sorbitol in pig feed to improve the lipid digestibility and weight gain of the animals.

In addition, KR Patent Laid-open No. 2007-31815 discloses use of an animal feed additive for livestock feed, comprising 95% of biotite containing vanadium and germanium and 5% of sulfur. Patents associated with animal feed additive comprising a variety of compositions have been published.

Meanwhile, fat is an essential nutrient for animals, which has a high energy value as compared to other nutrients and is the most expensive energy source per unit mass. Accordingly, an increase in fat utilization efficiency enables improvement of production efficiency and reduction of raw material prices of animal feed, thus cutting production costs.

KR Patent Laid-open No. 2004-7510 discloses an improved additive for animal feed. This patent suggests addition of polyoxyethylenesorbitan monooleate (Tween 60), polyoxyethylenesorbitan trioleate (Tween 80), polyoxyethylenesorbitan monostearate, alkyltrimethylammonium bromide or dodecyltrimethylammonium bromide, as nonionic surfactants, to an animal feed composition.

KR Patent Laid-open No. 2004-57438 relates to an immune booster composition for oral administration to fish. This patent suggests use of sodium dodecyl sulfate as an anionic surfactant to easily extract human gramilocyte colony stimulating factor from a yeast medium.

KR Patent Laid-open No. 2004-80172 relates to a microbial enzyme inducer and an animal feed composition comprising the same. This patent suggests use of Tween 60, Tween 80, Tween 80KD, Tween 85A, Tween 85B or Tween 100, as a nonionic surfactant for blocking supply of oxygen which inhibits growth of anaerobes and enabling higher fatty acids to serve as a source essential for growth of anaerobes.

Accordingly, as a result of intense and extended research to increase fat utilization efficiency in the bodies of animals, the inventors of the present invention discovered that sodium stearoyl-2-lactylate used as a hydrophilic emulsifier helps bile salt to emulsify fat in smaller particles and thereby improve fat absorption efficiency in the body. The present invention has been completed based on this discovery.

### [Disclosure]

### [Technical Problem]

Therefore, the present invention has been made in view of the above problems, and it is one object of the present invention to provide a bile salt adjuvant for animals to improve fat utilization efficiency in the bodies of animals and thereby improve production efficiency of livestock.

### [Technical Solution]

In accordance with one aspect of the present invention, the above and other objects can be accomplished by the provision of a bile salt adjuvant comprising sodium stearoyl-2-lactylate represented by Formula 1 below: 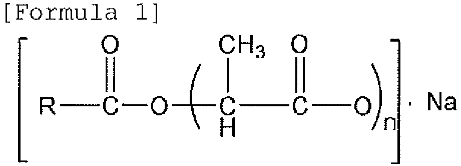 wherein R is C₁₇H₃₅ or C₁₅H₃₁, and n is an integer of 2. Further described is an animal feed comprising the sodium stearoyl-2-lactylate represented by Formula 1 as an active ingredient.

### [Effects of Invention]

The present invention provides the use of a compound represented by formula 1 as a bile salt adjuvant for an animal feed to increase digestion and utilization of fat present in an animal feed of a livestock animal wherein the animal feed comprises 5 to 30% by weight of crude protein; 2 to 20% by weight of crude fat; 2 to 20% by weight of crude fibre; 2 to 25% by weight of crude ash; 0.1 to 10% by weight of calcium; 0.1 to 5% by weight of lysine; 3 to 50% by weight of water; and 0.01 to 5% by weight of the compound of formula 1.

### [Best mode]

Hereinafter, the present invention will be described in more detail.

Neutral lipids contained in an animal feed composition cannot be directly absorbed by the body, instead being degraded by enzymes in the intestines and thus absorbed. The surface area of fat should be made as large as possible and emulsion particles (fat droplets) should be made as small as possible via bile salts which serves as a bioemulsifier in the intestines in order to obtain efficient enzymatic activity. As the size of fat droplets decreases, the surface area of fat increases and fat degradation is more rapidly and completely performed. In addition, the degraded fat acid is absorbed in the form of small oil droplets, called "micelle" in small intestine cells. At this time, as the size of micelles decreases, absorption efficiency increases.

The bile salt adjuvant for animals used for the present invention utilizes sodium stearoyl-2-lactylate (hereinafter, referred to as "SSL") as an additive which helps bile salt in the bodies of animals to convert fat present in animal feed into smaller fat droplets, thus widening the surface area, minimizing the size of micelles prior to absorption and thereby improving fat absorption efficiency in the body: 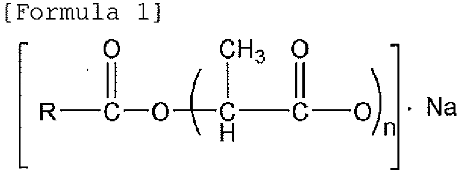 wherein R is C₁₇H₃₅ or C₁₅H₃₁, and n is an integer of 2.

SSL has an HLB value of about 20 and is thus considerably hydrophilic. SSL serves as an O/W (oil in water) type emulsifying agent which is readily dissolved in water and allows fat to be readily involved in the aqueous phase in the body, when used for animal feed compositions. For animal feed compositions, an emulsifying agent well dispersed in water is superior to an emulsifying agent well dispersed in oil, since metabolisms of digestive organs in the body use water as a medium under in vivo conditions. As a result, use of SSL enables an increase in fat decomposition efficiency and further improvement in digestion and utilization efficiency of fat.

SSL is commonly used as a food additive and is thus applicable to animal feed products (disclosed in GB 1581744 e.g.) The range applied to animal feed can be controlled by the amount of fats used for animal feed and SSL is preferably present in an amount 0.01 to 5% by weight, based on the total weight of the animal feed composition.

Any animal feed may be used so long as it is commonly used for livestock and preferred examples of animal feed include crude animal feed, concentrated animal feed, supplement animal feed, specific animal feed, protein animal feed, starch animal feed, fat animal feed, fibrous animal feed, inorganic animal feed, vitamin animal feed, antibiotic animal feed, amino acid animal feed and the like. These animal feed may be applied in a variety of forms such as a blend animal feed, ingredient animal feed, mixed animal feed and may contain SSL to lower fat.

Such a general animal feed is designed to have a suitable nutritional value by accurately determining a nutrient amount required for respective steps. The livestock is selected from pigs, chickens, ducks, quails, geese, pheasants, turkeys, cattle, dairy cows, horses, donkeys, sheep, goats, dogs, cats, rabbits and other cultured fish and shrimp.

Preferably, an animal feed composition comprising, as chemical ingredients, 5 to 30% by weight of crude protein, 2 to 20% by weight of crude fat, 2 to 20% by weight of crude fiber, 2 to 25% by weight of crude ash, 0.1 to 10% by weight of calcium, 0.1 to 5% by weight of phosphorus, 0.1 to 5% by weight of lysine and 3 to 50% by weight of water is used in the experimental example of the present invention.

The SSL is present in an amount of0.01 to 5% by weight, preferably 0.05 to 0.4% by weight, based on the total weight of the animal feed composition.

The use of SSL enables improvement in digestion and absorption efficiency of fats, growth promotion, improvement of qualities, and an increase in production yield. From the results of preferred experimental examples, it can be seen that, when 0.05% by weight of SSL is used, fat digestion percentage is increased to about 5 to 10% and ME is increased to about 100 kcal/kg.

In addition, the use of SSL enables an increase in digestion and absorption efficiency of fat and a decrease in the amount of fats used for animal feed, thus imparting cost savings (cost benefit) and being useful as low-fat animal feed.

The animal feed composition may further comprise an additive such as antibiotics, antibacterial agents, enzymes, organic acids, flavoring agents, sweetening agents, antioxidants and other functional substances, if necessary, in order to promote animal health, improve production efficiency and obtain positive effects for production of high-quality livestock.

### [Best Mode]

Now, the present invention will be described in more detail with reference to the following Examples.

### (Experimental Example 1) Fat emulsifying capacity test

In order to confirm emulsifying capacity of sodiumstearoyl-2-lactylate(hereinafter, referred to as "SSL") of the present invention, 75% water, 24.3% fat and 0.7% SSL (3% of the amount of fat used) were added and stirred. POE (20)-glyceride, mono-glyceride and lecithin were used as comparative examples. As a result, the SSL exhibited homogeneous dispersion, POE (20)-glyceride or lecithin underwent layer separation and oil-soluble mono-glyceride exhibited no emulsification.

### (Experimental Example 2) Test of effects on rat growth

Feed for animal tests purchased from Shin Chon Feed Co., Ltd, were prepared and various additives were added thereto to obtain a molded animal feed with a predetermined size.

4-week old male ICR rats with a weight of 11.5 to 13.5g were obtained from the laboratory animal center of Seoul National University, were divided into five groups (n=12) and maintained at a temperature of 23±2°C and a variety of specific animal feeds were administered thereto. At this time, animals of each group were treated with the following chemicals: (percentage (%) means% by weight)
- Control Group: animal feed + soybean oil 5%
- Experimental Group: animal feed+ soybean oil 5% + SSL 0.1% (HLB 20)
- Comparative Example 1: animal feed + soybean oil 5%+lecithin 0.1% (HLB 4)
- Comparative Example 2: animal feed + soybean oil 5%+ glycerin monocaprate 0.1% (HLB 7)
- Comparative Example 3: animal feed + soybean oil 5% + POE (20) sorbitan monolaurate 0.1% (HLB 16)

### (1) Weight variation of rats

Weights of each group were measured at an interval of 3 days before and after administration of animal feed, and the amount of animal feed administered was measured.

**TABLE 1**

| (g) | 0 day | 3 days | 6 days | 10 days | 13 days | 16 days | 19 days | 22 days | 25 days |
|---|---|---|---|---|---|---|---|---|---|
| Control group | 12.4± 1.32 | 15.3± 1.50 | 21.1± 1.50 | 24.9± 1.00 | 26.3± 1.43 | 29.1± 1.34 | 32,9± 2.14 | 34.7+ 2.98 | 35.3± 2.73 |
| Experi mental Group | 12.4± 1.56 | 15.9± 1.56 | 22.1± 1.90 | 25.8± 1.98 | 28.1± 1.65 | 30.8± 1.04 | 33.0± 1.63 | 35.7± 1.63 | 36.2± 1.68 |
| Compa rative Group 1 | 12.5± 1.16 | 15.6± 1.51 | 21.4± 1.26 | 25.6± 1.69 | 27.2± 2.09 | 29.2± 1.29 | 31.9± 2.49 | 33.9± 2.76 | 35.5± 2.98 |
| Compa rative Group 2 | 12,0± 1.45 | 15.4± 1.51 | 20.6± 1.74 | 24.8± 1.40 | 26.3± 1.15 | 29.0± 3.88 | 32.0± 1.33 | 33.7± 1.60 | 35.9± 1.77 |
| Compa rative Group3 | 12.9± 1.49 | 15.5± 1.74 | 21.2± 1.96 | 25.2± 2.22 | 26.5± 2.42 | 29.4± 1.96 | 32.3+ 2.32 | 33.6± 1.60 | 34.8± 2.41 |

As can be seen from Table 1 above, the Experimental Group, to which SSL was administered, exhibited the greatest increase in weight.

### (2) Evacuation of small intestinal propulsion in rats

Four rats of each group were weighed, 0.2 ml of BaSO₄suspension (BaSO₄:H₂O=1:1) was orally administered to the rats, the cervical spine of rats was dislocated and opened, after 30 minutes, the distance of BaSO₄ which moved in the small intestine was measured and small intestinal propulsion was evaluated as a percentage (%) of the total distance of small intestines.

**TABLE 2**

| Treatment | Control group | Experimental Group | Comparative Group 1 | Comparative Group 2 | Comparative Group 3 |
|---|---|---|---|---|---|
| Number of rats (%) | 41,6±4.9 | 34.1±5.1 | 40.8±3.9 | 36.9±4.5 | 38.6±4.1 |

The small intestinal propulsion tests are similar to diarrhea number tests. As the value decreases, the diarrhea number decreases.

As can be seen from Table 2 above, the control group and Comparative Example 1 containing lecithin exhibited the highest small intestinal propulsion, which indicates that the number of times of diarrhea in animals to which animal feed was administered is high. On the other hand, Experimental Group containing SSL exhibited the lowest small intestinal propulsion. These results indicate that SSL enables improvement in fat absorbance efficiency and has the potential to prevent alimentary diarrhea.

### (3) Measurement of serum biochemical index

At 15 hours after the final weight of the test animals was measured (25 days), blood was collected from supraorbital arrhythmia blood vessel plexus of the rats using a capillary tube, serum was separated, and the following biochemical indexes were measured using a kit.

GPT (Glutamate Pyruvate Transaminase, Reitman-Frankel method), GOT (glutamate oxaloacetate transaminase, Reitman-Frankel method), total protein (Biuret method), albumin (B.C.G. method), Cleantech TG-S (enzyme method)

**TABLE 3**

| | GPT (Kamen/mL) | GOT (Kamen/mL) | Total protein (g/L) | Albumin (g/L) | TG (mg/dL) |
|---|---|---|---|---|---|
| Control group | 46.3± 4.53 | 80.9± 8.38 | 55.9± 1.72 | 39.4± 0.97 | 143.9± 5.72 |
| Experimental Group | 33.8±1.98* | 63.9± 5.00* | 56.1± 0.98 | 39.8± 0.86 | 142.0± 9.98 |
| Comparative Group 1 | 42.7± 3.98 | 89.1± 12.80 | 58.2± 0.92 | 40.8± 1.11 | 148.9± 7.83 |
| Comparative Group2 | 58.9± 8.44 | 83.6± 5.15 | 56**.**7± 1.77 | 39.9± 0.91 | 154.8± 8.17 |
| Comparative Group 3 | 58.8± 18.91 | 115.6± 11.40^{#} | 56.0± 1.11 | 39.2± 0.66 | 157.1± 9.77 |
| Reference Value | 16-40 | 62-83 | 54-73 | 30-36 | 130-140 |
| N=8, * or #: P<0.05 (considerably distinguished from Control group) | | | | | |

As can be seen from Table 3 above, Experimental Group of the present invention exhibited 27% (GPT) and 21% (GOT) lower than those of Control groups (p<0.05) and Comparative Examples 1 to 3 exhibited an increase in GPT and GOT. In particular, Comparative Example 3 to which POE (20) sorbitan monolaurate was administered exhibited a considerable increase in GOT.

These results indicated that rats to which an animal feed containing SSL was administered for 4 weeks or longer exhibited considerable improvement in serum GPT and GOT.

### (4) Assay of fat level in feces

The rats of each group were starved in a metabolic cage, feces were collected over one night and the amount of fats contained therein was measured by soxhelt extraction.

**TABLE 4**

| Treatment (g) | 10 days after administration of animal feed | 25 days after administration of animal feed |
|---|---|---|
| Control group | 6.10 | 3.45 |
| Experimental Group | 3.80 | 3.02 |
| Comparative Group 1 | 5.66 | 3.26 |
| Comparative Group 2 | 4.95 | 3.57 |
| Comparative Group 3 | 5.56 | 3.39 |

As can be seen from Table 4, the Experimental Group exhibited the lowest fat level in the feces after animal feed was administered for 10 days, and the lowest fat level in feces after 25 days, which indicates that Experimental Group has high fat absorption in the body.

### (Experimental Example 3) Test of effects of SSL on pig growth

Experimental animals were grown at a breeding farm equipped with a mechanical ventilator in an Anseong laboratory farm of TS Corporation Co., Ltd., and the pig farm was designed to have a slat bottom and was provided with a single hole wet feeder to allow the animals to freely consume animal feed and water.

The tests were carried out on 24 pigs per group in one cycle at an interval of one week over a total period of three weeks using pigs (n=72 in total, Landrace x Yorkshire x Durce) including castrated pigs (n=36) and female pigs (n=36) having a mean weight of 29.97 kg.

Experimental animals were divided into 9 pens, each of which included four female pigs and four castrated pigs, based on base weight and gender, and 3 pens were randomly arranged for each treatment group (3x3 randomized complete block design). During test initiation and termination stages, the experimental animals were weighed and the amount of animal feed administered was measured weekly.

The mix ratio and ingredients of sample animal feed used for tests for the treatment groups and a control group designed to have a net energy of 2,320 kcal/kg are shown in Tables 5 and 6. The chemical ingredients in the sample animal feed were assayed using an AOAC (1990) method.

As compared to the control group, Experimental Group 1 contains 0.5% less animal fat (corn is used instead) and further contains 0.05% of SSL, and Experimental Group 2 contains 1.0% less animal fats (corn is used instead) and further contains 0.05% of SSL.

**TABLE 5**

| Content (% by weight) | Control group | Experimental Group 1 | Experimental Group 2 |
|---|---|---|---|
| Corn | 26.81 | 27.26 | 27.76 |
| Wheat | 10.00 | 10.00 | 10.00 |
| Cassava | 5.00 | 5.00 | 5.00 |
| Wheat bran | 9.42 | 9.42 | 9.42 |
| Corn germ meal | 4.00 | 4.00 | 4.00 |
| Soybean meal | 19.88 | 19.88 | 19.88 |
| Rapeseed meal | 3.00 | 3.00 | 3.00 |
| Dried distilled grains | 6.00 | 6.00 | 6.00 |
| Cookie by-product | 3.00 | 3.00 | 3.00 |
| Animal fat | 3.60 | 3.10 | 2,60 |
| Molasses | 6.00 | 6.00 | 6.00 |
| Liquid Lys(with HCl) | 0.28 | 0.28 | 0.28 |
| Liquid Choline | 0.06 | 0.06 | 0.06 |
| Limestone | 1.24 | 1.24 | 1.24 |
| Di-calcimn phosphate | 0.96 | 0.96 | 0.96 |
| Salt | 0.20 | 0.20 | 0.20 |
| Vitamin-mineral premix | 0.25 | 0.25 | 0.25 |
| Others (miscellaneous) | 0.30 | 0.30 | 0.30 |
| SSL | - | 0.05 | 0.05 |

**TABLE 6**

| Content (% by weight) | Control group | Experimental Group 1 | Experimental Group 2 |
|---|---|---|---|
| Moisture | 11.84 | 11.83 | 11.89 |
| Crude protein | 17.49 | 17.54 | 17.58 |
| Crude fat | 7.08 | 6.62 | 5.67 |
| Crude fiber | 4.31 | 4.30 | 4.31 |
| Crude ash | 5.81 | 5.79 | 5.80 |
| Calcium | 0.80 | 0.80 | 0.80 |
| Total phosphorous | 0.55 | 0.55 | 0.55 |
| Available phosphorus | 0.30 | 0.30 | 0.30 |
| Total lysine | 0.98 | 0.99 | 0.99 |
| Net energy, kcal | 2,320.00 | 2,295.00 | 2,270.00 |

Test results were obtained using the GLM procedures of the SAS statistical package (SAS, 1985), and final weight, and daily weight gain(ADG), amount of animal feed administered (ADFI) and required animal feed percentage were analyzed using Duncan's multiple range tests.

**TABLE 7**

| Items | Control group | Experimental Group 1 | (Experimental Group 2 |
|---|---|---|---|
| Initial weight (kg) | 29.80±4.70 | 29.90±4.70 | 30.20±5.20 |
| Final weight (kg) | 46.60±8.20 | 48.40±9.30 | 7.30±10.60 |
| Daily weight gain (ADG, kg) | 0.600±0.17 | 0.661±0.24 | 611±0.23 |
| Amount of animals feed administered (ADFI, kg) | 1.49±0.13 | 1.56±0.16 | 1.52±0.10 |
| Required animal feed percentage (FCR, %) | 2.48±0.79 | 2.36±0.67 | 2.49±0.43 |

As can be seen from Table 7 above, there was no large significant difference in final weight between the treatment groups and there was no significant difference in daily mean animal feed amount administered in a 95% reliable range, but Experimental Group 1 exhibited significant high daily mean animal feed amount administered in a 90% reliable range.

In terms of daily weight gain, Experimental Group 1 (which contains 0.5% less animal fat and additionally contains SSL, as compared to the control group) exhibited considerably high values as compared to the control group. In addition, Experimental group 2 (which contains 1.0% less animal fat and additionally contains SSL, as compared to the control group) also exhibited high daily weight gain as compared to the control group.

These results indicate that, when SSL is added, instead of a partial amount of fat, although the energy of the animal feed is decreased by reducing the level of fats added to the animal feed to 1.0%, as compared to the control group, digestion and utilization efficiency of administered fats are improved and there is thus no effect on daily weight gain. There was no statistical significant difference in required animal feed percentage in a treatment range (P>0.05, P>0.1), but Experimental Group 1 exhibited an increase in required animal feed percentage, as compared to control group. These results indicate that, although the level of fats in animal feed is decreased to 0.5%, as compared to the control group, in the case where an emulsifying agent is added, the digestion and utilization of administered fats are improved and required animal feed percentage can thus be improved.

As a result, when 0.05% of the SSL is added to animal feed, although the content of animal feed energy is decreased by decreasing the level of fats to 0.5%, as compared to the control group, production efficiency is improved (increase in daily administered animal feed amount, P<0.1). Although the level of fats is decreased to 1%, as compared to the control group, there is no effect on production efficiency (P>0.1).

### (Experimental Example 4) Effects of SSL on chicken growth

### Variation in growth ratio

Tests were carried out in a vertical integration farm including two poultry houses to grow 20,000 chickens in Hwasung, Kyunggi-do. Rainbow food was used as an animal feed before and after chicken growth. Tests were performed on 10,000 chickens placed in each poultry house. In order to confirm use of SSL instead of a partial amount of fats for chicken food on chicken growth, an initial chicken food, in which corn is added instead of 0.8% of cow fat and 0.05% of SSL is added, and a final chicken food, in which corn is added instead of 1.0% of cow fat and 0.05% of SSL is added, were prepared, and these initial and final chicken foods were administered to chickens to set Experimental Group. The results thus obtained are shown in Table 8.

**TABLE 8**

| Items | Control group | Experimental Group |
|---|---|---|
| Mean weight gain (kg) | 1.51 | 1.55 |
| Growing percentage (%) | 94.3 | 95.6 |
| Required animal feed percentage (FCR) | 1.80 | 1.78 |

As can be seen from Table 8, Experimental Group to which SSL is administered exhibited an increase in weight in spite of a decrease in required animal feed percentage. This result means that SSL contained in animal feed causes improved digestion and availability of fats in animal feed and results in improvement of required percentage of animal feed.

### [Industrial Applicability]

The bile salt adjuvant for animals used for the present invention is used for a variety of animals, livestock, poultry and the like.

## Claims

1. Use of a compound represented by the following Formula 1 as a bile salt adjuvant for an animal feed to increase digestion and utilization of fat present in an animal feed of a livestock animal wherein the animal feed comprises 5 to 30% by weight of crude protein; 2 to 20% by weight of crude fat; 2 to 20% by weight of crude fiber; 2 to 25% by weight of crude ash; 0.1 to 10% by weight of calcium; 0.1 to 5% by weight of lysine; 3 to 50% by weight of water; and 0.01 to 5% by weight of the compound of Formula 1 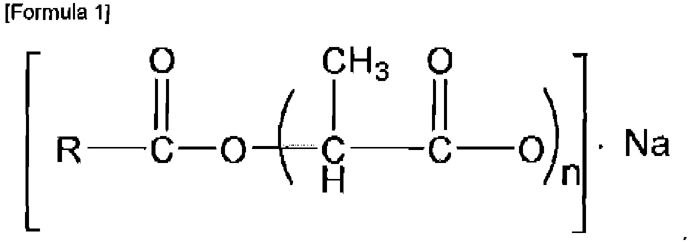 wherein R is C₁₇H₃₅ or C₁₅H₃₁., and n is an integer of 2.

2. The use according to claim 1, wherein the livestock animal is selected from at least one of pigs, chickens, ducks, quails, geese, pheasants, turkeys, cattle, milk cows, horses, donkeys, sheep, goats, dogs, cats, rabbits, cultured fish and shrimp.

3. A method for reducing the amount of fat required to be used in animal feed by increasing digestion and utilization of fat present in an animal feed of a livestock animal through feeding the animal with the animal feed containing a compound represented by the following Formula 1 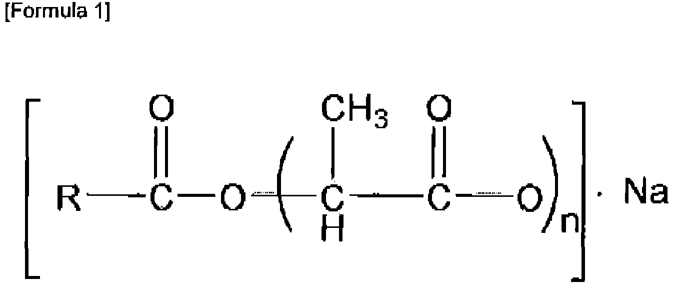 wherein R is C17H35 or G15H31, and n is an integer of 2, as a bile salt
adjuvant in amount of 0.01 to 5% by weight based on the total weight of the
animal feed.

4. The method according to claim 3, wherein the livestock animal is selected from at least one of pigs, chickens, ducks, quails, geese, pheasants, turkeys, cattle, milk cows, horses, donkeys,
sheep, goats, dogs, cats, rabbits, cultured fish and shrimp.

5. The method according to claim 3, wherein the animal feed comprises 5 to 30% by weight of crude protein; 2 to 20% by weight of crude fat; 2 to 20% by weight of crude fiber; 2 to 25% by weight of crude ash; 0.1 to 10% by weight of calcium; 0.1 to 5% by weight of lysine; and 3 to 50% by weight of water.

## Patentansprüche

1. Verwendung einer Verbindung dargestellt durch die folgende Formel 1 als ein Gallensalz-Adjuvans für eine Tiernahrung, um die Verdauung und die Verwertung von Fett, das in einer Tiernahrung eines Viehbestands-Tieres vorhanden ist, zu erhöhen, worin die Tiernahrung 5 bis 30 Gewichtsprozent unverarbeitetes Protein; 2 bis 20 Gewichtsprozent unverarbeitetes Fett; 2 bis 20 Gewichtsprozent unverarbeitete Ballaststoffe; 2 bis 25 Gewichtsprozent unverarbeitete Asche; 0,1 bis 10 Gewichtsprozent Kalzium; 0,1 bis 5 Gewichtsprozent Lysin; 3 bis 50 Gewichtsprozent Wasser; und 0,01 bis 5 Gewichtsprozent der Verbindung der Formel 1 umfasst 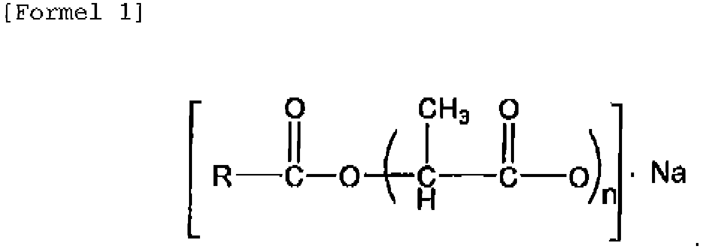 worin R C₁₇H₃₅ oder C₁₅H₃₁ ist, und n ist eine ganze Zahl von 2.

2. Die Verwendung gemäß Anspruch 1, worin das Viehbestands-Tier gewählt ist aus mindestens einem von Schweinen, Hühnern, Enten, Wachteln, Gänsen, Fasanen, Truthähnen, Rindern, Milchkühen, Pferden, Eseln, Schafen, Ziegen, Hunden, Katzen, Kaninchen, Kulturfischen und -garnelen.

3. Ein Verfahren zur Verminderung der Menge an erforderlich zu verwendendem Fett in Tiernahrung durch Erhöhen der Verdauung und der Verwertung von Fett, das in einer Tiernahrung eines Viehbestands-Tieres vorhanden ist, durch Füttern des Tieres mit der Tiernahrung, die eine Verbindung dargestellt durch die folgende Formel 1, enthält 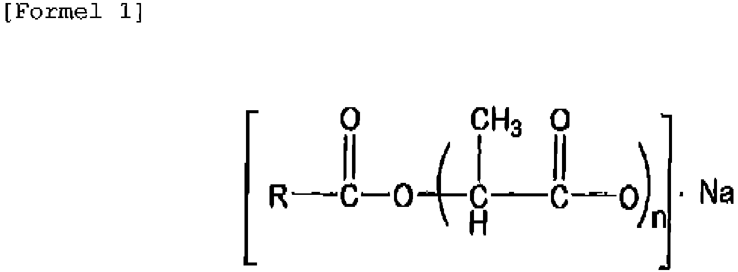 worin R C17H35 oder C15H31 ist, und n ist eine ganze Zahl von 2, als ein Gallensalz-Adjuvans in einer Menge von 0,01 bis 5 Gewichtsprozent basierend auf dem Gesamtgewicht der Tiernahrung.

4. Das Verfahren gemäß Anspruch 3, worin das Viehbestands-Tier gewählt ist aus mindestens einem vom Schweinen, Hühnern, Enten, Wachteln, Gänsen, Fasanen, Truthähnen, Rindern, Milchkühen, Pferden, Eseln, Schafen, Ziegen, Hunden, Katzen, Kaninchen, Kulturfischen und -garnelen.

5. Das Verfahren gemäß Anspruch 3, worin die Tiernahrung 5 bis 30 Gewichtsprozent unverarbeitetes Protein; 2 bis 20 Gewichtsprozent unverarbeitetes Fett; 2 bis 20 Gewichtsprozent unverarbeitete Ballaststoffe; 2 bis 25 Gewichtsprozent unverarbeitete Asche; 0**,**1 bis 10 Gewichtsprozent Kalzium; 0,1 bis 5% Gewichtsprozent Lysin; und 3 bis 50 Gewichtsprozent Wasser umfasst.

## Revendications

1. Utilisation d'un composé représenté par la formule 1 suivante en tant qu'adjuvant de type sel biliaire pour un aliment pour animaux, pour accroître la digestion et l'utilisation par un animal d'élevage de matières grasses présentes dans un aliment pour animaux, l'aliment pour animaux comprenant 5 à 30 % en poids de protéine brute ; 2 à 20 % en poids de matières grasses brutes ; 2 à 20 % en poids de fibres brutes ; 2 à 25 % en poids de cendres brutes ; 0,1 à 10 % en poids de calcium ; 0,1 à 5% en poids de lysine ; 3 à 50 % en poids d'eau ; et 0,01 à 5 % en poids du composé de formule I 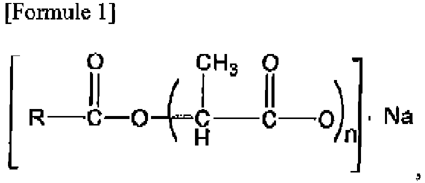 dans laquelle R est un groupe de formule C₁₇H₃₅ ou C₁₅H₃₁, et n est un nombre entier égal à 2.

2. Utilisation selon la revendication 1, dans laquelle l'animal d'élevage est choisi parmi au moins un des porcins, poulets, canards, cailles, oies, faisans, dindes, boeufs, vaches laitières, chevaux, ânes, ovins, caprins, chiens, chats, lapins, poissons et crevettes d'aquaculture.

3. Procédé pour la réduction de la quantité de matières grasses requises pour être utilisées dans un aliment pour animaux, par accroissement de la digestion et de l'utilisation par un animal d'élevage de matières grasses présentes dans un aliment pour animaux, par alimentation de l'animal avec l'aliment pour animaux contentant un composé représenté par la formule 1 suivante 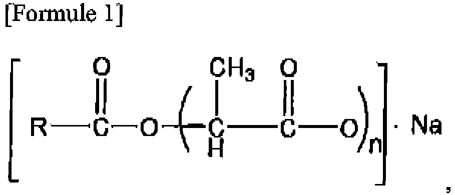 dans laquelle R est un groupe de formule C₁₇H₃₅ ou C₁₅H₃₁, et n est un nombre entier égal à 2, en tant qu'adjuvant de type sel biliaire, en une quantité de 0,01 à 5 % en poids, par rapport au poids total de l'aliment pour animaux.

4. Procédé selon la revendication 3, dans lequel l'animal d'élevage est choisi parmi au moins un des porcins, poulets, canards, cailles, oies, faisans, dindes, boeufs, vaches laitières, chevaux, ânes, ovins, caprins, chiens, chats, lapins, poissons et crevettes d'aquaculture.

5. Procédé selon la revendication 3, dans lequel l'aliment pour animaux comprend 5 à 30 % en poids de protéine bruts ; 2 à 20 % en poids de matières grasses brutes ; 2 à 20 % en poids de fibres brutes ; 2 à 25 % en poids de cendres brutes ; 0,1 à 10 % en poids de calcium ; 0,1 à 5 % en poids de lysine ; et 3 à 50 % en poids d'eau.
